# EUROPEAN PATENT APPLICATION

(11) **EP 2 388 626 A2**
(43) Date of publication of application: **23.11.2011**
(21) Application number: 11003988.0
(22) Date of filing: 13.05.2011
(51) Int. Cl.: G02B 6/00, G02B 23/24, A61B 1/06

(54) **Illumination device**

(30) Priority: 19.05.2010 JP 2010115216
(71) Applicant: OLYMPUS CORPORATION, Shibuya-ku Tokyo 151-0072 (JP)
(72) Inventor: Furuta, Koichiro, Tokyo 151-0072 (JP)
(74) Representative: Schmidt, Steffen J.

(57) **Abstract**

To provide an illumination device which can emit a white light beam having a high luminance and having high color rendering properties at the time of a white light mode and which can also emit an illumination light beam having a high luminance at the time of a special light mode. There is provided an illumination device 100 configured by including: LEDs 1 to 12 which are arranged in a circular ring shape at intervals in the circumferential direction of the circular ring and are arranged with the optical axis thereof directed in the radially inward direction of the circular ring; a light guide member 30 which guides an illumination light beam emitted from each of the LEDs 1 to 12 in the direction along the central axis L of the circular ring; a motor 35 which rotationally drives the light guide member 30 about the central axis L; and a control section 36 which lights the LEDs 1 to 12 in a pulse-like manner in synchronization with the motor 35, the illumination device 100 being configured such that at least one of the LEDs 1 to 12 includes light emitting body units 25, 26 and 27 having emission spectra different from each other, and dichroic prisms 28 and 29 for synthesizing the light beams emitted from the light emitting body units 25, 26 and 27 and for emitting the synthesized light in the optical axis direction.

## Description

### BACKGROUND OF THE INVENTION

### 1. FIELD OF THE INVENTION

The present invention relates to an illumination device including a plurality of light sources.

This application is based on Japanese Patent Application No. 2010-115216, the content of which is incorporated herein by reference.

### 2. DESCRIPTION OF RELATED ART

Conventionally, there is known an illumination device which emits a white light beam having a high luminance in such a manner that R, G, B light sources, such as LEDs, arranged in a circular ring shape and respectively emitting light beams of R, G and B colors are successively lighted at high output power in a pulse-like manner, and that the illumination light beams emitted from each of the light sources are superimposed by rotating a light guide rod in synchronization with the lighting period of the light sources (see, for example, Japanese Unexamined Patent Application, Publication No. 2008-102304 (hereinafter referred to as JP 2008-102304).

### BRIEF SUMMARY OF THE INVENTION

However, in the illumination device described in JP 2008-102304, the wavelength band of the white light beam is formed by three bands of R, G and B colors, and hence wavelength missing occurs between the respective bands. Therefore, a problem may arise when the white light beam is used as an illumination light beam requiring high color rendering properties.

Further, at the time of a special light mode for emitting a light beam having a specific wavelength band, only the LEDs necessary for emitting the special light beam are lighted, and the LEDs unnecessary for emitting the special light beam are unlighted. For this reason, the light amount corresponding to the unlighted LEDs is not included in the light amount of the special light beam, and hence the light amount of the special light beam is reduced by the light amount corresponding to the unlighted LEDs.

The present invention has been made in view of the above described circumstances. An object of the present invention is to provide an illumination device which can emit a white light beam having a high luminance and high color rendering properties at the time of a white light mode, and which can also emit an illumination light beam having a high luminance at the time of a special light mode.

To achieve the above-described object, the present invention adopts the following means.

The present invention adopts an illumination device configured by including: a plurality of light sources which are arranged in a circular ring shape at intervals in the circumferential direction of the circular ring and are arranged with the optical axis thereof directed in the radially inward direction of the circular ring; a light guide section which has an incident surface arranged on the outside in the radial direction of the circular ring for receiving an illumination light beam emitted from each of the light sources and guides the illumination light beam incident on the incident surface in the direction along the central axis of the circular ring; a rotating section which rotationally drives the light guide section about the central axis; and a control section which lights the plurality of light sources in a pulse-like manner in synchronization with the rotating section, the illumination device being configured such that at least one of the plurality of light sources includes a plurality of light emitting bodies having emission spectra different from each other, and a light synthesizing section which synthesizes the light beams emitted from the plurality of light emitting bodies and emits the synthesized light beam in the direction of the optical axis.

According to the present invention, the light guide section having the incident surface arranged on the outside in the radial direction of the circular ring is rotationally driven about the central axis of the circular ring by the rotating section. In this case, the plurality of light sources arranged in the circular ring shape at intervals in the circumferential direction of the circular ring are lighted by the control section in a pulse-like manner in synchronization with the rotating section. Specifically, each of the light sources, which faces the incident surface of the light guide section, is successively lighted by the control section in the pulse-like manner. Thereby, the high intensity illumination light beam emitted from each of the light sources can be continuously made incident on the incident surface of the light guide section, so as to be superimposed and emitted in the direction along the central axis of the circular ring.

In this case, at least one of the plurality of light sources includes the plurality of light emitting bodies having emission spectra different from each other, and the light synthesizing section which synthesizes the light beams emitted from the plurality of light emitting bodies and emits the synthesized light beam in the radially inward direction of the circular ring. The illumination light beam emitted from the light source having such configuration is a light beam formed by synthesizing light beams having emission spectra different from each other. Therefore, when a white light beam is to be illuminated, a white light beam without wavelength missing and having high color rendering properties can be illuminated by superimposing the illumination light beams emitted from these light sources.

Further, when a light beam having a specific wavelength band is to be illuminated, only the light emitting body emitting a light beam having a necessary wavelength band may be lighted, instead that the whole light source is unlighted. Thereby, also when a light beam having a specific wavelength band is to be illuminated, the illumination light beams from each of the light sources can be superimposed and emitted, and hence the intensity of the illumination light beam can be improved.

In the above-described invention, the control section may also be configured to select, according to an illumination mode, the light emitting body to be lighted.

With this configuration, for example, at the time of a white light mode for emitting a white light beam, a white light beam without wavelength missing and having high color rendering properties can be emitted. Further, at the time of a special light mode for emitting a light beam having a specific wavelength band, illumination light beams, which are emitted from each of the light sources by lighting, in each of the light sources, only the light emitting body for emitting a light beam having a necessary wavelength band, can be superimposed and emitted, and hence an illumination light beam having a high luminance can be emitted.

In the above-described invention, the plurality of light emitting bodies may be respectively configured by light emitting bodies for emitting light beams of R, G and B components.

With this configuration, a white light beam can be emitted from each of the light sources by lighting all of the light emitting bodies for emitting light beams of R, G and B components, and by synthesizing the light beams of R, G and B components. Further, a light beam having a specific wavelength band can be emitted from each of the light sources by lighting one of the light emitting bodies for emitting light beams of R, G and B components.

In the above-described invention, it may also be configured such that the optical axis of one of the light emitting bodies is arranged in the radially inward direction, such that the optical axis of the other light emitting body is arranged perpendicularly to the radial direction, and such that the light synthesizing section transmits the light beam from the one light emitting body and reflects the light beam from the other light emitting body in the radially inward direction.

With this configuration, the light beam from the light emitting body (one light emitting body) having the optical axis arranged in the radially inward direction is transmitted through the light synthesizing section, and the light beam from the light emitting body (other light emitting body) having the optical axis arranged perpendicularly to the radial direction is reflected by the light synthesizing section in the radially inward direction. Thereby, the light beam from the light emitting body (one light emitting body) having the optical axis arranged in the radially inward direction and the light beam from the light emitting body (other light emitting body) having the optical axis arranged perpendicularly to the radial direction are synthesized and emitted in the radially inward direction.

In the above-described invention, it may also be configured such that a plurality of the other light emitting bodies are arranged side by side in the radial direction, and such that a plurality of the light synthesizing sections are arranged side by side in the radial direction.

With this configuration, the light beams from three or more light emitting bodies can be synthesized and emitted in the radially inward direction of the circular ring.

In the above-described invention, the light synthesizing section may be configured by one or more dichroic mirrors.

With this configuration, it is possible that the light synthesizing section can transmit the light beam from one light emitting body and reflect the light beam from the other light emitting body in the radially inward direction of the circular ring. Thereby, the light beam from the one light emitting body and the light beam from the other light emitting body are synthesized and emitted in the radially inward direction.

In the above-described invention, it may also be configured such that the peak wavelength of the emission spectrum of each of the light beams emitted from the plurality of light emitting bodies and synthesized by the light synthesizing section is separated from the peak wavelength of the other emission spectrum adjacent to the emission spectrum by at least approximately the sum of the half-value widths of each of the emission spectra.

With this configuration, the emission spectra of the light beams, which are emitted from the plurality of light emitting bodies and synthesized by the light synthesizing section, can be prevented from overlapping with each other, that is, prevented from being too close to each other, and hence the color rendering properties of the illumination light beam emitted from each of the light sources can be improved.

In the above-described invention, it may also be configured such that a light guide member for collimating and guiding the light beam emitted from each of the light sources is arranged between each of the plurality of light sources and the light guide section.

With this configuration, the light beam emitted from each of the light sources is collimated by the light guide member so as to be made incident on the incident surface of the light guide section, and hence the emission efficiency of the light source can be improved.

In the above-described invention, it may also be configured such that the light guide member has a shape having a cross sectional area increased from the incident surface for receiving the illumination light beam from the light source toward the emission surface for emitting the illumination light beam incident on the incident surface.

When the light guide member is formed into the tapered rod shape in this way, the incident illumination light beam can be collimated, so that the NA (Numerical Aperture) can be improved and the brightness of the illumination light beam can be increased.

The present invention provides the effects that a white light beam having a high luminance and high color rendering properties can be emitted at the time of a white light mode, and that an illumination light beam having a high luminance can be also emitted at the time of a special light mode.

### BRIEF DESCRIPTION OF THE SEVERAL VIEWS OF THE DRAWINGS

FIG. 1 is a plan view showing a schematic configuration of an illumination device according to an embodiment of the present invention;
FIG. 2 is a longitudinal sectional view of the illumination device of FIG. 1;
FIG. 3 is a table showing light emitting states of each illumination mode in the illumination device of FIG. 1;
FIG. 4 is a timing chart of each LED at the time of a white light mode in the illumination device of FIG. 1;
FIG. 5 is a timing chart of each LED at the time of special light mode 1 in the illumination device of FIG. 1;
FIG. 6 is a timing chart of each LED at the time of special light mode 2 in the illumination device of FIG. 1;
FIG. 7 is a timing chart of each LED at the time of special light mode 3 in the illumination device of FIG. 1;
FIG. 8 is a graph showing emission spectra of respective light emitting body units in the illumination device of FIG. 1;
FIG. 9 is a graph showing a spectrum of light beams superimposed in the illumination device of FIG. 1;
FIG. 10 is a graph showing a transmission characteristic of a dichroic prism in the illumination device of FIG. 1;
FIG. 11 is a graph showing spectra of illumination light beams synthesized by the dichroic prism of FIG. 10;
FIG. 12 is a plan view showing a schematic configuration of a conventional illumination device;
FIG. 13 is a graph showing spectra of light beams superimposed in the illumination device of FIG. 12;
FIG. 14 is a graph showing a transmission characteristic of a dichroic prism in the illumination device of FIG. 12; and
FIG. 15 is a graph showing spectra of illumination light beams synthesized by the dichroic prism of FIG. 14.

### DETAILED DESCRIPTION OF THE INVENTION

In the following, an illumination device according to an embodiment of the present invention will be described with reference to the accompanying drawings.

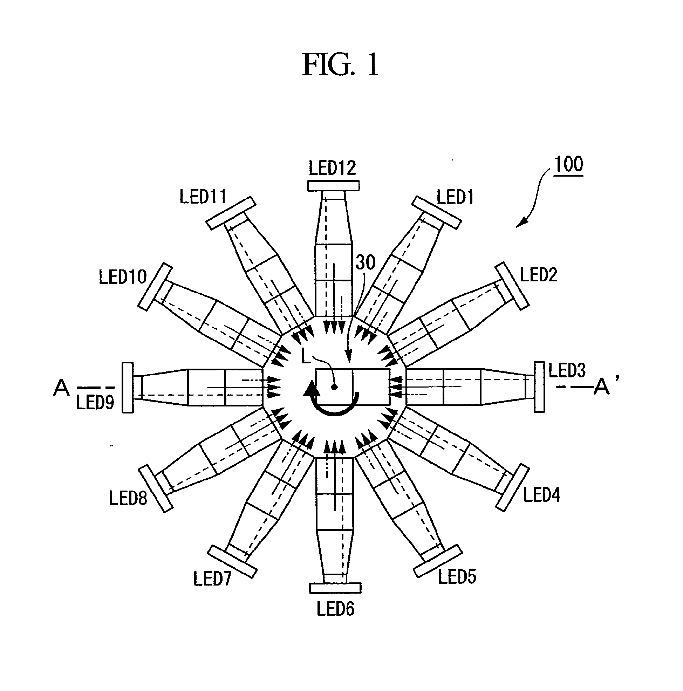

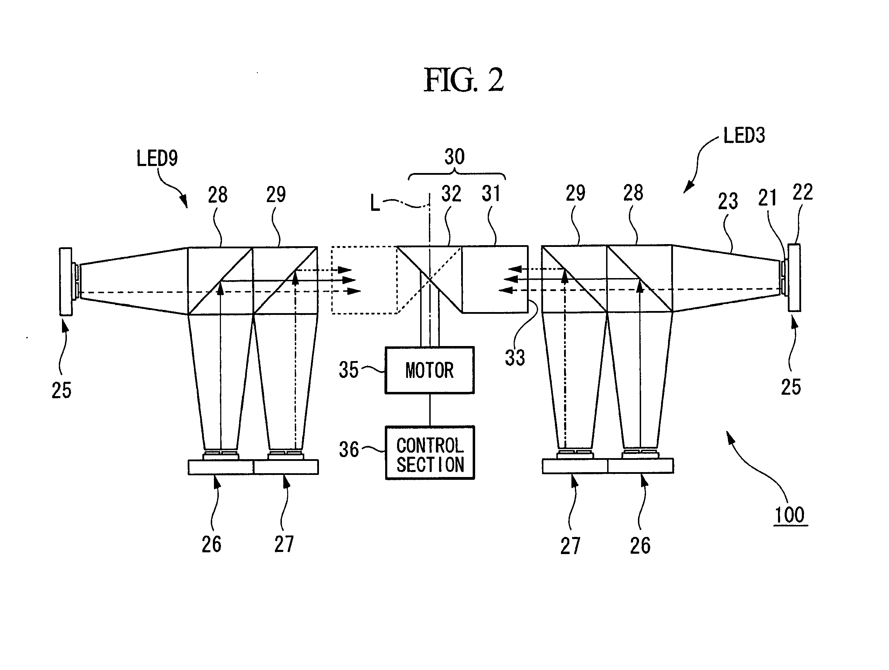

FIG. 1 is a plan view showing a schematic configuration of an illumination device 100 of the present embodiment, and FIG. 2 is a longitudinal sectional view along the line A-A' in FIG. 1.

As shown in FIG. 1 and FIG. 2, the illumination device 100 includes a plurality of LEDs (light sources) 1 to 12 arranged side by side in a circular ring shape, a light guide member (light guide section) 30 arranged in the radially inward portion of the circular ring shape formed by arranging the LEDs 1 to 12, a motor (rotating section) 35 for rotating the light guide member 30 about the central axis L of the circular ring, and a control section 36 for controlling the LEDs 1 to 12 and the motor 35.

The LEDs 1 to 12 are arranged at predetermined intervals in the circumferential direction of the circular ring and are arranged with the optical axis thereof directed in the radially inward direction of the circular ring, and are supplied with drive current from a power source (not shown) to thereby emit illumination light beams in the radially inward direction of the circular ring.

Since the LEDs 1 to 12 have the same configuration, the LED 3 is taken as a representative example and is described with reference to FIG. 2.

As shown in FIG. 2, the LED 3 includes a light emitting body unit 25 for emitting an illumination light beam of an R component, a light emitting body unit 26 for emitting an illumination light beam of a G component, a light emitting body unit 27 for emitting an illumination light beam of a B component, a first dichroic prism (light synthesizing section) 28 for synthesizing the illumination light beam emitted from the light emitting body unit 25 and the illumination light beam emitted from the light emitting body unit 26, and a second dichroic prism (light synthesizing section) 29 for synthesizing the illumination light beam from the first dichroic prism 28 and the illumination light beam emitted from the light emitting body unit 27.

The light emitting body unit 25 is arranged with the optical axis thereof directed in the radially inward direction of the circular ring formed by the LEDs 1 to 12. Each of the light emitting body units 26 and 27 is arranged with the optical axis thereof directed in the direction (direction along the central axis L) perpendicular to the radial direction. Here, the light emitting body units 26 and 27 have the same configuration except that the emission spectra of the light emitting body units 26 and 27 are different from each other.

The light emitting body unit 25 includes, for example, a light emitting body 21, such as an LED, a substrate 22 for fixing the light emitting body 21, and a light guide rod 23 for guiding the illumination light beam emitted from the light emitting body 21.

The light guide rod 23 is a tapered rod for guiding the illumination light beam from the light emitting body 21, and has a shape in which the cross sectional area of the rod is increased from the incident surface for receiving the illumination light beam from the light emitting body 21 toward the emission surface for emitting the illumination light beam incident on the incident surface.

When the light guide rod 23 is formed into such a tapered rod shape, the incident illumination light beam can be collimated, so that the NA (Numerical Aperture) can be improved and the brightness of the illumination light beam can be increased.

The first dichroic prism 28 is arranged at the position where the optical axis of the light emitting body unit 25 and the optical axis of the light emitting body unit 26 cross each other. The first dichroic prism 28 transmits the illumination light beam emitted from the light emitting body unit 25 and reflects in the radially inward direction the illumination light beam emitted from the light emitting body unit 26. Thereby, the first dichroic prism 28 synthesize the illumination light beam emitted from the light emitting body unit 25 and the illumination light beam emitted from the light emitting body unit 26, and emits the synthesized light in the radially inward direction.

The second dichroic prism 29 is arranged at the position where the optical axis of the light emitting body unit 25 and the optical axis of the light emitting body unit 27 cross each other. The second dichroic prism 29 transmits the synthesized light beam synthesized by the first dichroic prism 28, and reflects in the radially inward direction the illumination light beam emitted from the light emitting body unit 27. Thereby, the second dichroic prism 29 synthesizes the synthesized light beam from the first dichroic prism 28 and the illumination light beam emitted from the light emitting body unit 27, and guides the synthesized light beam in the radially inward direction.

With the above-described configuration, the LED 3 synthesizes the illumination light beams emitted from each of the light emitting body units 25, 26 and 27 having mutually different emission spectra, and emits the synthesized light beam toward the light guide member 30.

Each of the LEDs 1 and 2, and the LEDs 4 to 12 has the same configuration as that of the LED 3, and synthesizes the illumination light beams emitted from each of the light emitting body units 25, 26 and 27, so as to emit the synthesized light beam toward the light guide member 30.

As shown in FIG. 2, the light guide member 30 includes a light guide rod 31 which has an incident surface 33 for receiving the illumination light beam emitted from each of the LEDs 1 to 12, and which guides the illumination light beam incident on the incident surface 33 in the radially inward direction of the circular ring, and a reflecting prism 32 which reflects, in the direction along the central axis L of the circular ring, the illumination light beam guided by the light guide rod 31. With this configuration, the light guide member 30 emits, in the direction along the central axis L, the illumination light beam emitted in the radially inward direction from the LEDs 1 to 12.

The rotary shaft of the motor 35 is connected to the reflecting prism 32 and is arranged on the central axis L. Thus, the light guide rod 31 and the reflecting prism 32 are integrally driven rotationally about the central axis L by the motor 35.

The control section 36 lights the LEDs 1 to 12 in a pulse-like manner in synchronization with the motor 35. Specifically, the control section 36 successively lights in a pulse-like manner the LED facing the incident surface 33 of the light guide rod 31.

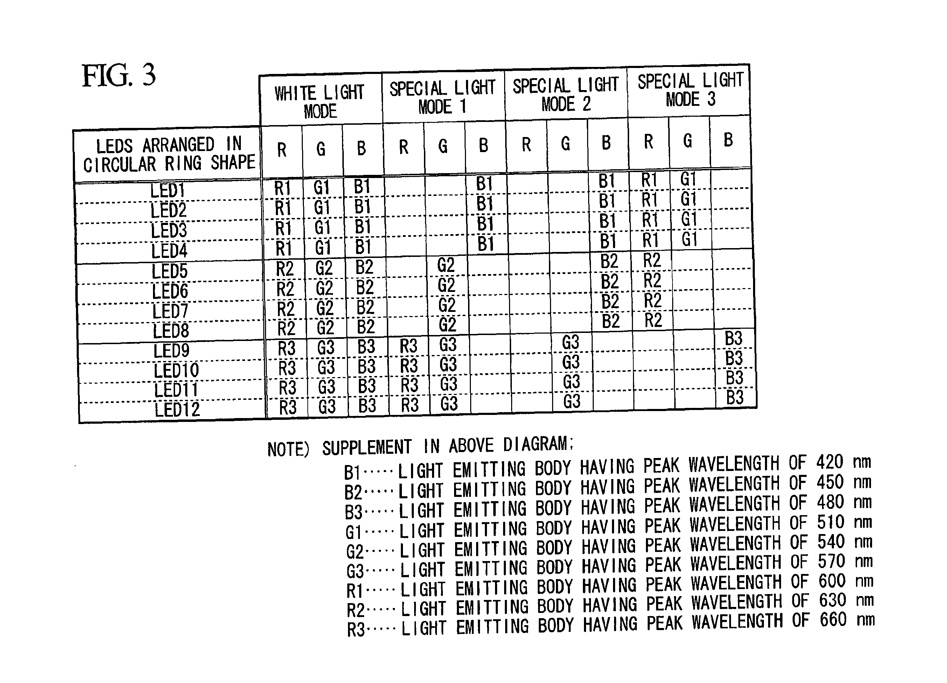

Further, according to an illumination mode, the control section 36 selects the light emitting body unit to be lighted. Specifically, for example, as shown in FIG. 3, the control section 36 has four illumination modes of a white light mode, a special light mode 1, a special light mode 2, and a special light mode 3.

In the example shown in FIG. 3, the LEDs 1 to 4 are configured such that the light emitting body unit 25 emits an illumination light beam of an R1 component, such that the light emitting body unit 26 emits an illumination light beam of a G1 component, and such that the light emitting body unit 27 emits an illumination light beam of a B1 component. Further, the LEDs 5 to 8 are configured such that the light emitting body unit 25 emits an illumination light beam of an R2 component, such that the light emitting body unit 26 emits an illumination light beam of a G2 component, and such that the light emitting body unit 27 emits an illumination light beam of a B2 component. Further, the LEDs 9 to 12 are configured such that the light emitting body unit 25 emits an illumination light beam of an R3 component, such that the light emitting body unit 26 emits an illumination light beam of a G3 component, and such that the light emitting body unit 27 emits an illumination light beam of a B3 component.

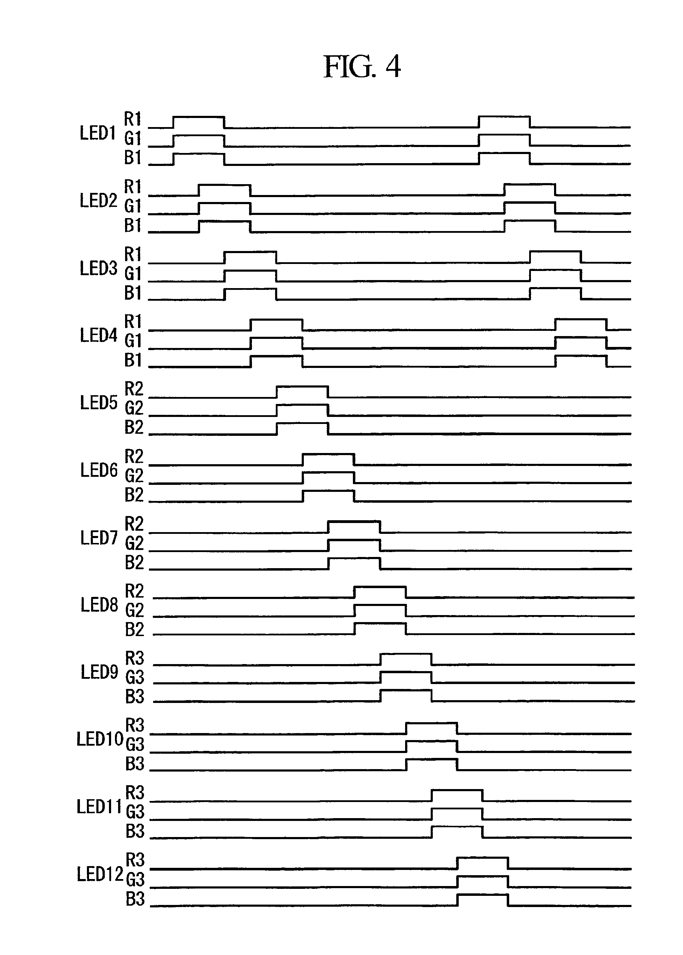

In this configuration, the control section 36 lights all of the light emitting body units of the LEDs 1 to 12 in the white light mode. In this case, as shown in FIG. 4, each of the light emitting body units is successively lighted in synchronization with the rotation of the light guide member 30 driven by the motor 35.

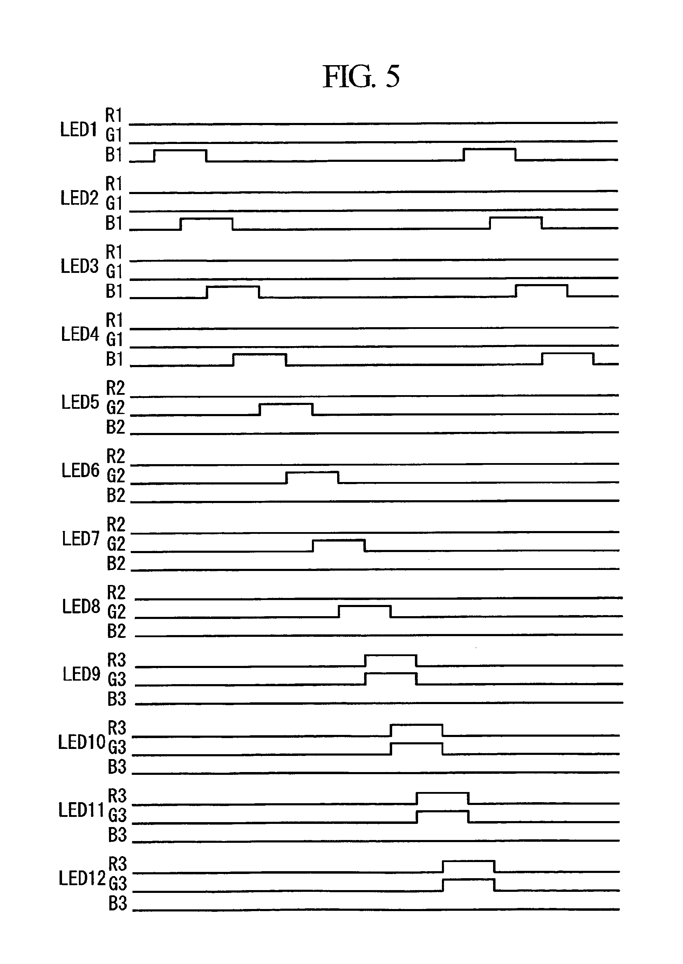

In the special light mode 1, the control section 36 lights only the light emitting body unit 27 of the LEDs 1 to 4, the light emitting body unit 26 of the LEDs 4 to 12, and the light emitting body unit 25 of the LEDs 9 to 12. In this case, as shown in FIG. 5, each of the light emitting body units selected as described above is successively lighted in synchronization with the rotation of the light guide member 30 driven by the motor 35.

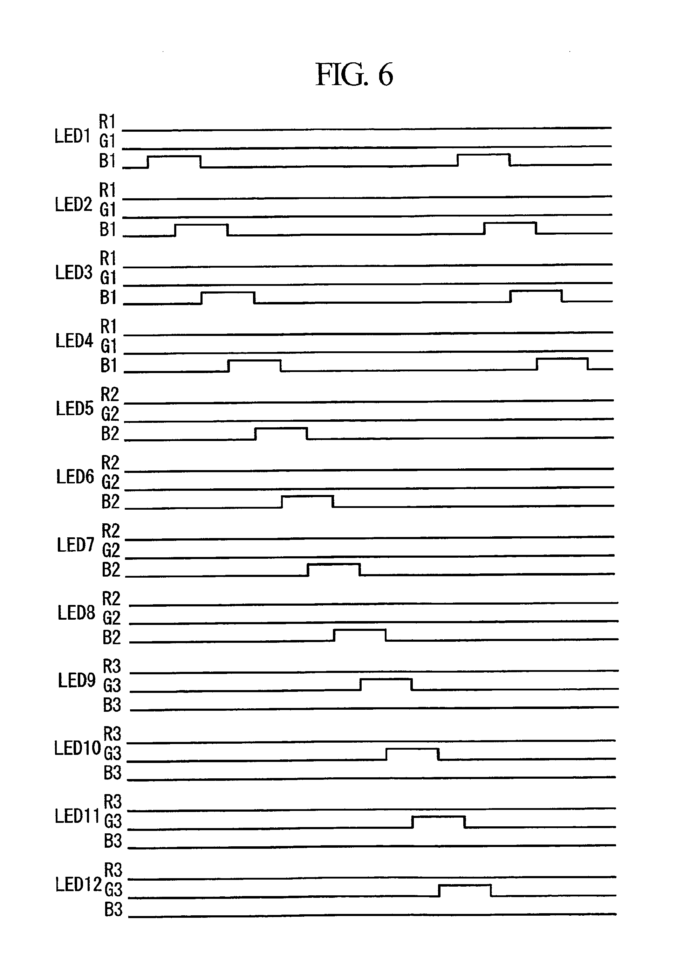

In the special light mode 2, the control section 36 lights only the light emitting body unit 27 of the LEDs 1 to 8, and the light emitting body unit 26 of the LEDs 9 to 12. In this case, as shown in FIG. 6, each of the light emitting body units selected as described above is successively lighted in synchronization with the rotation of the light guide member 30 driven by the motor 35.

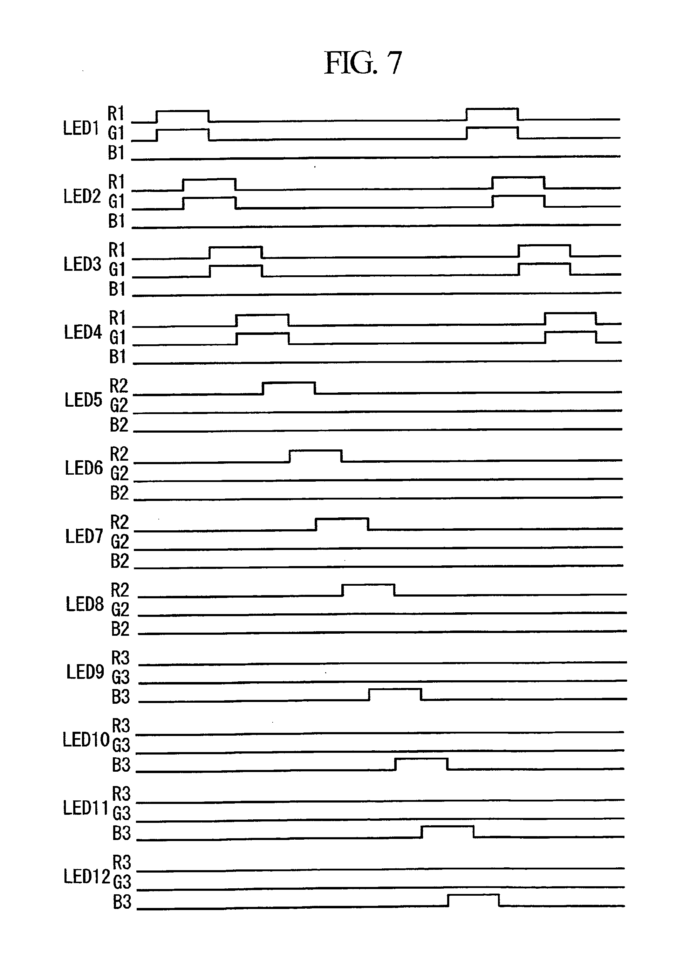

In the special light mode 3, the control section 36 lights only the light emitting body unit 25 and the light emitting body unit 26 of the LEDs 1 to 4, the light emitting body unit 25 of the LEDs 4 to 8, and the light emitting body unit 27 of the LEDs 9 to 12. In this case, as shown in FIG. 7, each of the light emitting body units selected as described above is successively lighted in synchronization with the rotation of the light guide member 30 driven by the motor 35.

The operation of the illumination device 100 which performs the above-described control will be described below.

When the illumination device 100 is started, the light guide member 30 is driven rotationally about the central axis L by the motor 35, and the LED facing the incident surface 33 of the light guide member 30 is successively lighted in a pulse-like manner. Thereby, illumination light beams emitted from each of the LEDs 1 to 12 and having a high intensity are continuously made incident on the incident surface 33 of the light guide member 30, so as to be superimposed and emitted toward an object to be illuminated.

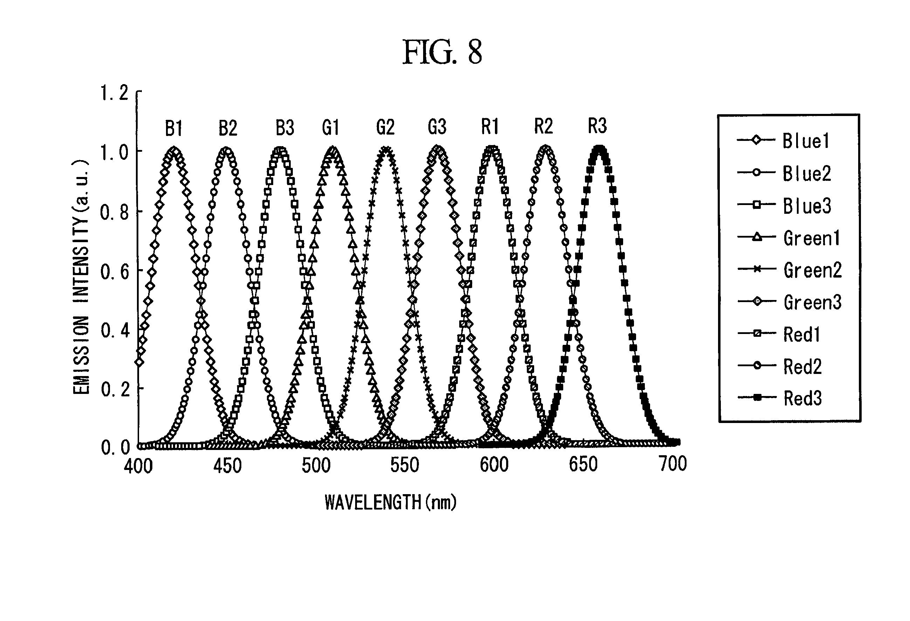

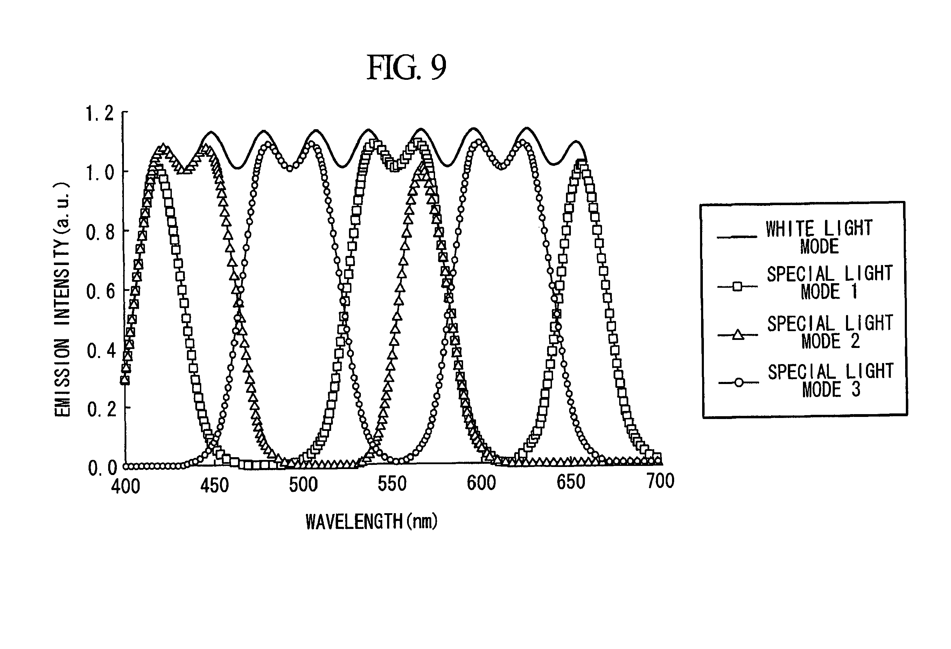

In this case, the illumination light beam emitted from each of the LEDs 1 to 12 is a light beam obtained by synthesizing the illumination light beams emitted from each of the light emitting body units. Further, the light emitting body unit to be lighted is selected by the control section 36 according to the illumination mode. Here, the light beam emitted from each of the light emitting body units in the LEDs 1 to 12 has a spectrum as shown in FIG. 8. When illumination light beams emitted from these light emitting body units are synthesized according to the illumination mode, an illumination light beam having different wavelength components can be emitted as shown in FIG. 9 from the light guide member 30 according to the illumination mode.

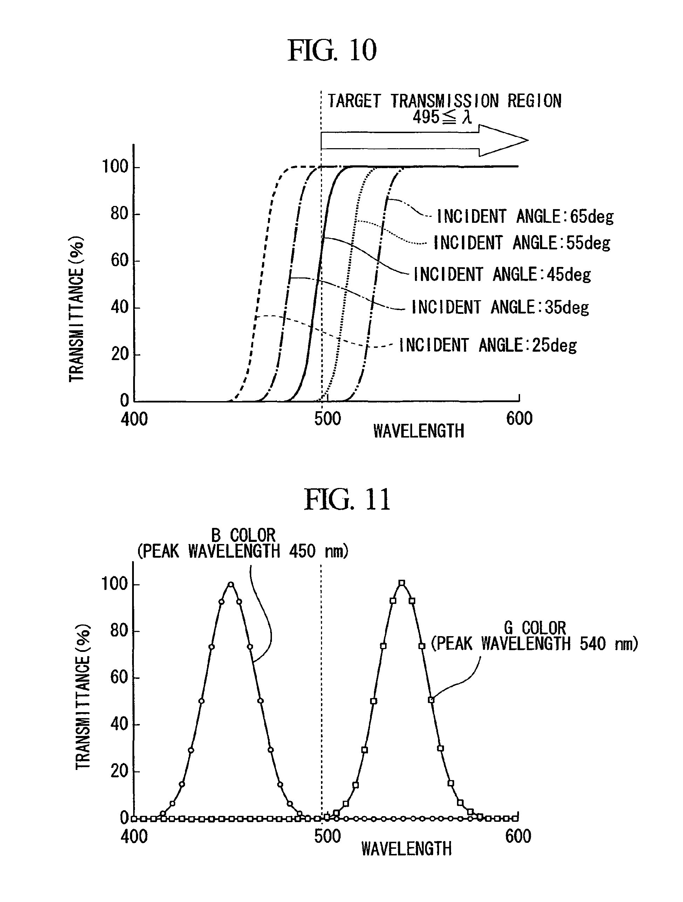

Further, each of the first dichroic prism 28 and the second dichroic prism 29 has a transmission characteristic (reflection characteristic) for transmitting a light beam in a predetermined wavelength band and for reflecting a light beam in a wavelength band other than the predetermined wavelength band. Here, the second dichroic prism 29 provided in the LEDs 5 to 8 is taken as an example, and the transmission characteristic (reflection characteristic) of the second dichroic prism 29 will be described with reference to FIG. 10 and FIG. 11.

In FIG. 10, the incident angle means an angle at which each of the illumination light beam is made incident on the second dichroic prism 29. In the present embodiment, since the optical axis of the light emitting body unit 25 is arranged in the radially inward direction of the circular ring, and since the optical axis of the light emitting body unit 25 is arranged perpendicularly to the optical axes of the light emitting body unit 26 and the light emitting body unit 27, the incident angle of each of the illumination light beams is 45°.

Further, as shown in FIG. 11, the peak wavelength of the illumination light beam of the B2 component emitted from the light emitting body unit 26 is 450 nm, and the peak wavelength of the illumination light beam of the G2 component emitted from the light emitting body unit 27 is 540 nm.

Therefore, as shown in FIG. 10, the transmission characteristic (reflection characteristic) of the second dichroic prism 29 provided in each of the LEDs 5 to 8 is set in the transmission region defined by the center value (495 nm) between the peak wavelengths of the illumination light beams of these components. Thereby, the second dichroic prism 29 provided in each of the LEDs 5 to 8 can reflect the illumination light beam of the B2 component (having the peak wavelength of 450 nm) emitted from the light emitting body unit 26, and can transmit the illumination light beam of the G2 component (having the peak wavelength of 540 nm) emitted from the light emitting body unit 27, so that the illumination light beams emitted from the light emitting body units can be synthesized and emitted in the radially inward direction without wasting the illumination light beams.

Similarly, when the transmission characteristic (reflection characteristic) of each of the other dichroic prisms 28 and 29 is set as described above, the illumination light beams made incident on each of the dichroic prisms 28 and 29 can be synthesized and emitted in the radially inward direction without wasting the illumination light beams.

Note that, in the illumination device 100 according to the present embodiment, the peak wavelength of the emission spectrum of each of the illumination light beams emitted from the light emitting body units 25, 26 and 27 and synthesized by the dichroic prisms 28 and 29 is preferably separated from the peak wavelength of the other emission spectrum adjacent to the emission spectrum by at least approximately the sum of the half-value widths of each of the emission spectra.

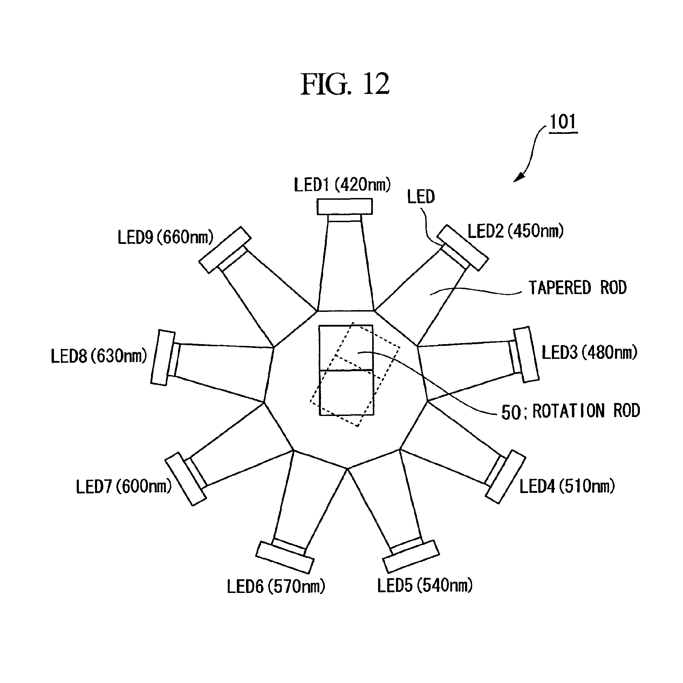

Here, as a comparison example, a conventional illumination device 101 will be described with reference to FIG. 12.

As shown in FIG. 12, the conventional illumination device 101 includes a plurality of LEDs 1 to 9 arranged side by side in a circular ring shape, a rotation rod 50 arranged in the radially inward portion of the circular ring shape formed by arranging the LEDs 1 to 9, and a motor (not shown) for rotating the rotation rod 50 about the central axis of the circular ring.

The conventional illumination device 101 is configured so as to emit a white light beam having a high luminance in such a manner that the LEDs 1 to 9 for respectively emitting light beams of R, G and B colors are successively lighted at high output power and that the illumination light beams from the LEDs 1 to 9 are superimposed by rotating the rotation rod 50 in synchronization of the lighting period of the LEDs.

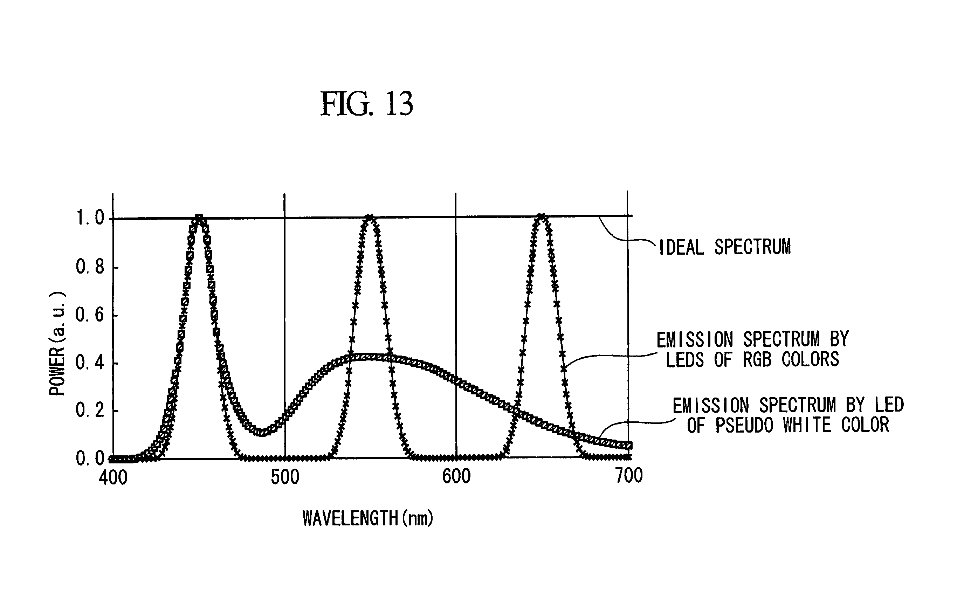

However, in the conventional illumination device 101, the white color is formed by using three bands of the R, G and B colors as shown in FIG. 13, and hence wavelength missing occurs between the respective bands. Therefore, when the white light beam is used as an illumination light beam requiring high color rendering properties, a problem may arise. On the other hand, when an LED for emitting a pseudo-white light beam is used as each of the LEDs 1 to 9, an illumination light beam whose intensity is high in a specific wavelength band is emitted as shown in FIG. 13, and hence a problem may also arise similarly to the above described case.

Further, in the conventional illumination device 101, at the time of a special light mode for emitting a light beam having a specific wavelength band, only LEDs necessary for emitting the special light beam are lighted, and the unnecessary LEDs are not lighted. For this reason, the light amount of the special light beam does not include the light amount corresponding to the unlighted LEDs, and hence the light amount of the special light beam is reduced by the light amount corresponding to the unlighted LEDs.

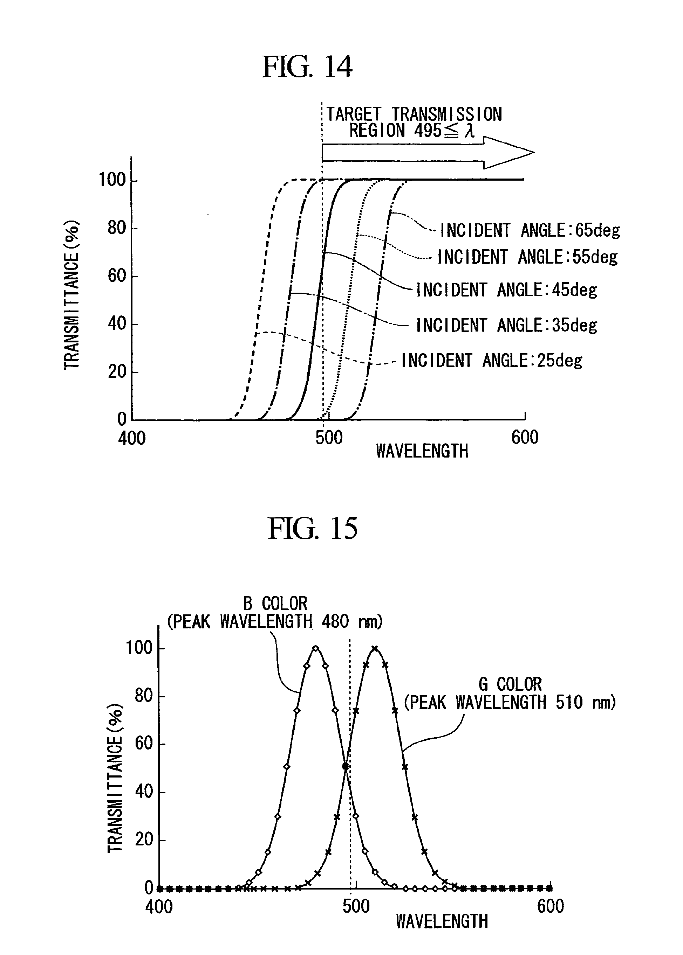

Further, in the conventional illumination device 101, when the peak wavelengths of the illumination light beams are set close to each other in order to improve the color rendering properties of the white light beam, the wavelength regions of the illumination light beams overlap with each other in a plurality of wavelength regions as shown in FIG. 15. In this case, it is conceivable to provide a dichroic prism (mirror) at the rotation rod 50. However, in this case, even when the transmission characteristic of the dichroic prism is set in the transmission region defined by the center value between the peak wavelengths of the illumination light beams as shown in FIG. 14, the reflected light beam and the transmitted light beam mixedly exist, so that the portion of the illumination light beams which portion is not synthesized is increased. This results in a problem that the light amount of the synthesized light beam is reduced.

On the other hand, in the illumination device 100 according to the present embodiment, each of the LEDs 1 to 12 includes the light emitting body units 25, 26 and 27 having different emission spectra, and the dichroic prisms 28 and 29 for synthesizing the light beams emitted from each of the light emitting body units 25, 26 and 27 and for emitting the synthesized light beam in the radially inward direction of the circular ring. That is, the illumination light beam emitted from each of the LEDs 1 to 12 is a light beam obtained by synthesizing light beams having mutually different emission spectra. Therefore, as shown in FIG. 9, when a white light beam is emitted (at the time of a white light mode), a white light beam without wavelength missing and having high color rendering properties can be illuminated by superimposing the illumination light beams emitted from the LEDs 1 to 12.

Further, in the illumination device 100 according to the present embodiment, when a light beam having a specific wavelength band is emitted as shown in FIG. 9, only the light emitting body unit for emitting the light beam having the necessary wavelength band may be lighted, instead that the whole LED is unlighted. Thereby, even when the light beam having the specific wavelength band is emitted, the illumination light beams from each of the LEDs can be superimposed and emitted, so that the intensity of the illumination light beam can be improved.

Further, in the illumination device 100 according to the present embodiment, the dichroic prisms 28 and 29 for synthesizing light beams of different wavelength components are provided in each of the LEDs as shown in FIG. 2, and hence each of the dichroic prisms 28 and 29 can be formed to have a transmission characteristic suitable for synthesizing the illumination light beams from each of the light emitting body units. Thereby, the illumination light beams from each of the light emitting body units can be synthesized and emitted in the radially inward direction without wasting the illumination light beams, so that the illumination light beams having high luminances can be superimposed and emitted by the light guide member 30.

Further, as described above, in the illumination device 100 according to the present embodiment, the peak wavelength of the emission spectrum of each of the light beams, which are emitted from the light emitting body units 25, 26 and 27 and synthesized by the dichroic prisms 28 and 29, are separated from the peak wavelength of the other emission spectrum adjacent to the emission spectrum by at least approximately the sum of the half-value widths of each of the emission spectra.

With this configuration, the emission spectra of the light beams, which are emitted from the light emitting body units 25, 26 and 27 and which are synthesized by the dichroic prisms 28 and 29, can be prevented from overlapping with each other, that is, can be prevented from being too close to each other, so that the color rendering properties of the illumination light beams emitted from each of the LEDs can be improved.

In the above, an embodiment according to the present invention has been described in detail with reference to the accompanying drawings. However, a specific configuration is not limited to the embodiment, and a design change, and the like, within the scope of the present invention is also included in the specific configuration.

For example, in the illumination device 100 according to the present embodiment, an example provided with twelve LEDs (LED 1 to LED 12) is described, but the number of LEDs is not limited. However, the light guide efficiency is increased as the number of LEDs arranged in the circular ring shape is increased. This is because, since the installation angle between adjacent LEDs arranged in the circular ring shape is reduced as the number of LEDs is increased, it is possible to suppress the reduction in light guide efficiency which is caused while the light guide member 30 is positioned between the adjacent LEDs during the rotation of the light guide member 30. Note that in the actual design, the cost is also taken into consideration, and hence a smaller number of LEDs is preferred.

Further, the number of the light emitting body units provided in each of the LEDs is not limited to three. Basically, it is preferred that each of the LEDs has three light emitting body units, but there may be an LED having one, or two light emitting body units, or an LED having four or more light emitting body units. Note that, when only one light emitting body unit is provided in an LED, the light synthesizing section (dichroic prisms 28 and 29) is not necessary. Further, the number of light emitting body units may be changed for each LED.

Further, in the illumination device 100 according to the present embodiment, a dichroic mirror having the same transmission characteristic may also be used in place of each of the dichroic prisms 28 and 29.

### DESCRIPTION OF SYMBOLS

- 1 to 12: LED (light source)
- 21: light emitting body
- 22: substrate
- 23: light guide rod (light guide member)
- 25, 26, 27: light emitting body unit
- 28: first dichroic prism (light synthesizing section)
- 29: second dichroic prism (light synthesizing section)
- 30: light guide member (light guide section)
- 31: light guide rod
- 32: reflecting prism
- 35: motor (rotating section)
- 36: control section
- 100: illumination device
- L: central axis

## Claims

1. An illumination device comprising:
a plurality of light sources which are arranged in a circular ring shape at intervals in the circumferential direction of the circular ring and are arranged with the optical axis thereof directed in the radially inward direction of the circular ring;
a light guide section which has an incident surface arranged on the outside in the radial direction of the circular ring for receiving an illumination light beam emitted from each of the light sources and guides the illumination light beam incident on the incident surface in the direction along the central axis of the circular ring;
a rotating section which rotationally drives the light guide section about the central axis; and
a control section which lights the plurality of light sources in a pulse-like manner in synchronization with the rotation section,
wherein at least one of the plurality of light sources includes
a plurality of light emitting bodies having emission spectra different from each other, and
a light synthesizing section which synthesizes the light beams emitted from the plurality of light emitting bodies and emits the synthesized light in the optical axis direction.

2. The illumination device according to claim 1, wherein, according to an illumination mode, the control section selects the light emitting body to be lighted.

3. The illumination device according to claim 1, wherein the plurality of light emitting bodies are light emitting bodies which emit light beams of R, B and G components, respectively.

4. The illumination device according to claim 1,
wherein the optical axis of one of the light emitting bodies is arranged in the radially inward direction and the optical axis of the other light emitting body is arranged perpendicularly to the radial direction, and
wherein the light synthesizing section transmits the light beam from the one light emitting body and reflects the light beam from the other light emitting body in the radially inward direction.

5. The illumination device according to claim 4,
wherein a plurality of the other light emitting bodies are arranged side by side in the radial direction, and
wherein a plurality of the light synthesizing sections are arranged side by side in the radial direction.

6. The illumination device according to claim 4, wherein the light synthesizing section is configured by one or more dichroic mirrors.

7. The illumination device according to claim 1, wherein the peak wavelength of the emission spectrum of each of the light beams emitted from the plurality of light emitting bodies and synthesized by the light synthesizing section is separated from the peak wavelength of the other emission spectrum adjacent to the emission spectrum by at least approximately the sum of the half-value widths of each of the emission spectra.

8. The illumination device according to claim 1, wherein a light guide member for collimating and guiding the light beam emitted from each of the light sources is arranged between each of the plurality of light sources and the light guide section.

9. The illumination device according to claim 8, wherein the light guide member has a shape having a cross sectional area increased from the incident surface for receiving the illumination light beam from the light source toward the emission surface for emitting the illumination light beam incident on the incident surface.
